# EUROPÄISCHE PATENTANMELDUNG

(11) **EP 4 492 393 A1**
(43) Veröffentlichungstag der Anmeldung: **15.01.2025**
(21) Anmeldenummer: 23184826.8
(22) Anmeldetag: 11.07.2023
(51) Int. Cl.: G16H 20/17, G16H 40/67, H04L 67/55, G06F 3/023

(54) **VERFAHREN UND SYSTEM ZUR AUTOMATISCHEN FERNPROGRAMMIERUNG VON INFUSIONSPUMPEN**

(71) Anmelder: B. Braun Melsungen AG, 34212 Melsungen (DE)
(72) Erfinder: Kever, Felix, 34125 Kassel (DE)
(74) Vertreter: Tergau & Walkenhorst Intellectual Property GmbH

(57) **Zusammenfassung**

Verfahren zur automatischen Fernprogrammierung von Infusionspumpen mittels Infusionsaufträgen, die in/von einem Patientendaten Management-System einer medizinischen Einrichtung bereitgestellt werden, wobei in einem Verifikationsschritt ein an einem Eingabemittel eingegebener Kurzbestätigungscode mit einem Kurzbestätigungscode, der einem von der Infusionspumpe empfangenen Infusionsauftrag unmittelbar zugeordnet ist, verglichen wird.

## Beschreibung

Die Erfindung betrifft ein Verfahren und ein System zur automatischen Fernprogrammierung von Infusionspumpen mittels Infusionsaufträgen, die in/von einem Patientendaten Management-System einer medizinischen Einrichtung bereitgestellt werden.

Stand der Technik ist der in Teilen der Welt schon seit vielen Jahren eingesetzte AutoProgramming- oder BCMA-Workflow, wobei BCMA für Barcode Medication Administration steht, also in etwa Barcode-gestützte Medikamentengabe.

Vorausgesetzt wird ein Electronic Medical Record (EMR) System oder auch Patientendaten Management-System in einer medizinischen Einrichtung wie beispielsweise einem Krankenhaus, einem Pflegeheim oder dergleichen. Ausgehend von einem im EMR-System erstellten Medikationsauftrag für einen bestimmten Patienten, mit einer bestimmten Medikation sowie genauen Vorgaben zur Applikation werden von diesem System zunächst maschinenlesbare (Barcode-) Labels für den Medikationscontainer sowie - falls nicht bereits vorhanden - den Patienten erstellt.

Am Bettplatz des Patienten scannt die Krankenschwester das mit Barcode versehene Patientenarmband, den in der Vorbereitung mit dem entsprechenden Barcode versehenen Medikationsbehälter und zuletzt die ebenfalls mit einem Barcode versehene Infusionspumpe, welche zum Einsatz kommen soll, mit einem Scanner, welcher mit dem EMR-System verbunden ist. Dort werden die vor Ort gescannte Patienten-ID und Medikations-ID nun mit dem vorliegenden Medikationsauftrag abgeglichen (richtiger Patient? richtige Medikation? etc.) und bei erfolgreicher Prüfung an die zuvor ebenfalls gescannte Infusionspumpe gesendet.

Auf der Pumpe findet eine weitere Prüfung statt, indem die Medikation und die Vorgaben zur Applikation (Dosis- oder Flussrate, zu infundierendes Volumen, Limits, ...) mit den in der Medikamentenbibliothek der Infusionspumpe gespeicherten Daten und Vorgaben abgeglichen werden.

Schließlich zeigt die Infusionspumpe die empfangenen Daten zur finalen Bestätigung auf dem Display an; danach kann die Infusionstherapie gestartet werden. Die laufenden Infusionsdaten werden dann inklusive der Auftrags-ID zurück an das EMR-System gesendet, so dass sie automatisch dem entsprechenden Medikationsauftrag und/oder der elektronischen Patientenakte des Patienten zugeordnet werden können.

Die Nachteile dieses Verfahrens lassen sich wie folgt zusammenfassen:
- Ein Barcodescanner sowie eine Benutzerschnittstelle zum EMR-System müssen für jede zu startende Infusionstherapie am Bettplatz verfügbar und betriebsbereit sein oder von jeder Krankenschwester mitgeführt werden.
- Das Durchlaufen der jeweils nötigen Scanvorgänge am Patientenbett sowie der jeweiligen Bestätigungen im EMR-System ist allein schon vom Ablauf her zeitaufwändig.
- Durch die physischen Abstände zwischen der Benutzerschnittstelle des EMR-Systems, dem Patientenarmband, dem Medikationsbehälter und der Infusionspumpe ist unter Umständen ein mehrfacher räumlicher Wechsel erforderlich, welcher den Arbeitsablauf verkompliziert und weiteren Zeitaufwand verursacht.
- Die oben genannten Punkte führen zu einer geringen Akzeptanz sowohl bei den Workflow-Verantwortlichen als auch bei den Krankenschwestern / dem Pflegepersonal, insbesondere wenn es um eine Neueinführung des BCMA-Workflows geht.
- Der wesentliche Teil des BCMA-Workflows (inklusive der Scanner-basierten Verifikation des Infusionsauftrags) ist auf Seiten des EMR-Systems zu implementieren; das Infusionspumpen-System erhält lediglich den fertig geprüften Infusionsauftrag zur Anzeige auf / Ausführung durch die Pumpe. Dies hat dazu geführt, dass aktuell lediglich wenige EMR-Systeme den BCMA-Workflow unterstützen.
- Das alles kann letztlich resultieren im Auftreten von Medikations- und Behandlungsfehlern zu Lasten von Patienten, die mit dem BCMA-Workflow sicher hätten vermieden werden können.

Aufgabe der Erfindung ist es, einen Workflow (Verfahren) und ein zugehöriges System (Vorrichtung) zur einfachen, gegen Verwechslungen abgesicherten Fernprogrammierung von Infusionspumpen durch ein EMR-System bereitzustellen, der/das die Nachteile des Standes der Technik zumindest teilweise vermeidet oder abmildert und insbesondere ohne aufwendige Scanvorgänge durch das Pflegepersonal am Patientenbett auskommt.

Die genannte Aufgabe wird erfindungsgemäß gelöst durch ein Verfahren nach den Merkmalen des Anspruchs 1 sowie durch ein System (Vorrichtung) mit den Merkmalen des Anspruchs 10.

Demnach ist ein Verfahren zur automatischen Fernprogrammierung von Infusionspumpen vorgesehen mittels Infusionsaufträgen, die in/von einem Patientendaten Management-System einer medizinischen Einrichtung bereitgestellt werden, wobei die medizinische Einrichtung
- eine Anzahl von Betten aufweist, die jeweils mit einem Patienten belegbar sind,
- eine Anzahl von Infusionspumpen aufweist, die jeweils an ein Backend eines Infusionspumpensystems angeschlossen sind,
   wobei
- das Patientendaten Management-System über eine erste elektronische Schnittstelle Infusionsaufträge zur Zwischenspeicherung an das Backend übermittelt,
- die jeweilige Infusionspumpe über eine zweite elektronische Schnittstelle zwischengespeicherte Infusionsaufträge vom Backend empfängt,
   wobei ferner
- dem jeweiligen Infusionsauftrag ein von einem Menschen lesbarer Kurzbestätigungscode als eindeutige Kennung unmittelbar zugeordnet und bei Übermittlungen mitgegeben wird,
- dem jeweiligen Infusionsauftrag ein Medikationscontainer zugeordnet wird, der mit einem den zugeordneten Kurzbestätigungscode enthaltenden Informationsträger ausgestattet ist,
- die jeweilige Infusionspumpe eine Aufnahme oder einen Anschluss für einen der Medikationscontainer aufweist,
- der jeweiligen Infusionspumpe ein Eingabemittel zur Eingabe des zugehörigen, dem Informationsträger des Medikationscontainers entnehmbaren Kurzbestätigungscodes zugeordnet ist,
wobei schließlich
in einem Verifikationsschritt ein am Eingabemittel eingegebener Kurzbestätigungscode mit dem Kurzbestätigungscode, der dem von der Infusionspumpe empfangenen Infusionsauftrag unmittelbar zugeordnet ist, verglichen wird, und wobei nur bei Übereinstimmung der Kurzbestätigungscodes der Infusionsauftrag von der Infusionspumpe ausgeführt wird oder auf Aufforderung ausführbar ist.

Der Begriff "Bett" oder "Bettplatz" ist in der vorliegenden Anmeldung vorzugsweise in einem weiten Sinne zu verstehen und beinhaltet jeglichen Infusionsplatz, an dem einem Patienten oder Kunden eine Infusion verabreicht werden kann.

Damit steht ein deutlich vereinfachter Workflow zur abgesicherten Fernprogrammierung von Infusionspumpen durch ein EMR-System zur Verfügung. Durch den Wegfall von bis zu drei Scan-Vorgängen und die Verlagerung sämtlicher Nutzerinteraktion auf die Infusionspumpe wird der gesamte Ablauf wesentlich schneller, nutzerfreundlicher und ist zudem leichter zu implementieren - unter Beibehaltung sämtlicher Benefits des klassischen AutoProgramming/BCMA-Workflows, insbesondere unter Ausschluss von Fehlzuordnungen der Medikation zum Patient.

Dabei ist es in einer Grundversion erforderlich, dass das EMR-System eine (aktuelle) Zuordnung von Betten zu Patienten enthält, und/oder das das Backend eine Zuordnung von Betten zu Infusionspumpen enthält. Beides kann beispielsweise in Gestalt von Zuordnungstabellen implementiert sein.

Mit anderen Worten ist in der Grundversion eine fehlerfreie und zum Zeitpunkt der Übertragung des Infusionsauftrages aktuelle Zuordnung von Patient und Bettplatz (im EMR-System) sowie von Bettplatz und Pumpe (im Infusionspumpen-System) eine zwingende Voraussetzung für das beschriebene Verfahren.

Eine Alternative wäre die zusätzliche Eingabe eines zweiten, patientenbezogenen Codes an der Pumpe (d. h. eines bevorzugt ebenfalls durch alphanumerische Codierung stark verkürzten Codes), welcher vom EMR-System erstellt und verwaltet wird und den Patienten identifiziert. Dieser Code wäre entweder zusätzlich auf dem Patientenarmband aufgedruckt oder würde die üblicherweise recht lange und rein numerische Patienten-ID ersetzen. In der Konsequenz wäre dies wäre ein zusätzlicher Eingabeschritt der Pflegekraft, andererseits entfällt dabei die Notwendigkeit der Bettplatzzuordnung der Pumpe.

Zweckmäßigerweise erfolgt der Vergleich der Kurzbestätigungscodes dezentral, außerhalb des EMR und außerhalb des Backends, und zwar bevorzugt in einer in die jeweilige Infusionspumpe integrierten Steuerung. Der Vergleich könnte alternativ oder zusätzlich aber auch im Backend / im IOM stattfinden.

Weiterhin ist es bevorzugt, dass die jeweilige Infusionspumpe via Pull Kommunikation über die zweite elektronische Schnittstelle ihr zugeordnete Infusionsaufträge vom Backend abfragt und empfängt, während das Patientendaten Management-System bevorzugt Infusionsaufträge via Push-Kommunikation über die erste elektronische Schnittstelle an das Backend übermittelt. Im erstgenannten Fall handelt es sich typischerweise um eine indirekte Zuordnung, da die Medikationsaufträge bevorzugt nicht explizit einer Pumpe zugeordnet sind, sondern dem Patienten, dem die Pumpe wiederum indirekt (d. h. über das Bett) zugordnet ist.

In einer vorteilhaften Variante wird dem jeweiligen Infusionsauftrag ein Gültigkeitszeitraum oder eine Gültigkeitsdauer zugeordnet und im Verifikationsschritt berücksichtigt. Dadurch kann auch bei einer vergleichsweise großen medizinischen Einrichtung mit vielen Infusionen ein relativ kurzer Kurzbestätigungsode ausreichend sein.

Wenn der Kurzbestätigungsode ein alphanumerischer Code ist, ist er vom Pflegepersonal besonders einfach zu handhaben und über das Eingabefeld der Infusionspumpe einzugeben. Der Begriff "alphanumerisch" soll vorliegend sowohl die Möglichkeit eines rein numerischen Codes (insbesondere aus den Ziffern 0 bis 9) als auch eines rein buchstabenbasieren Codes (vorzugsweise aus den lateinischen Großbuchstaben A bis Z, ggf. ohne O und/oder ohne I) als auch eines aus den genannten Bestandteilen gemischten bzw. kombinierten Codes beinhalten. Ein dreistelliger alphanumerischer Code kann im zuletzt genannten Fall rund 42.000 Varianten abbilden und ist damit - auch mit Blick auf die begrenzte Gültigkeitsdauer - in aller Regel ausreichend.

Anstelle oder zusätzlich zu einer Eingabe über ein Eingabefeld / Touchdisplay an der Infusionspumpe kann eine Eingabe des Codes über ein der Infusionspumpe zugeordnetes Eingabemittel, z. B. ein Eingabefeld am Rack, oder über ein Mobile Device (Smartphone, Tablet oder dergleichen) erfolgen.

In einem bevorzugten einfachen Fall handelt es sich bei dem Informationsträger um eine ablesbare Beschriftung, insbesondere eine aufgedruckte Beschriftung, beispielsweise in Gestalt eines mit dem Kurzbestätigungscode beschrifteten Aufklebers, der auf den Medikationscontainer geklebt wird, oder in Gestalt eines beschrifteten Zettels, Streifens, Labels oder sonstigen beschrifteten Mediums, der/das mit dem Medikationsträger verbunden ist. Es ist aber auch denkbar, dass der Informationsträger den Kurzbestätigungscode in anderer Form enthält und beispielsweis maschinell / automatisiert ausgelesen um dem Eingabemittel zugeführt wird.

Die für das Verfahren genannten Merkmale und Vorteile übertragen sich analog auf die Vorrichtung.

Das im Rahmen der vorliegenden Erfindung beschriebene Systemkonzept / der Workflow der verifizierten Verwaltung von Infusionsvorgängen oder auch Verified Infusion Administration (VIA) erfüllt den gleichen Zweck wie der bekannte BCMA-Workflow, ist jedoch wesentlich nutzerfreundlicher und damit einfacher in der Implementierung sowie der täglichen Anwendung.

Die wesentlichen Unterschiede sind die Steuerung des gesamten Prozesses über die Infusionspumpe durch kombinierte Push/Pull-Kommunikation sowie der Wegfall sämtlicher Scanvorgänge - einerseits durch Nutzung bereits vorhandener Daten, andererseits durch Einsatz eines kurzen, von Menschen handhabbaren Kurzbestätigungscodes oder Verifizierungscodes. Das VIA-Konzept erfordert dabei einige Neuerungen sowohl auf Seiten des EMR-Systems als auch auf Seiten des Infusionspumpen-Systems.

### VIA-bedingte Neuerungen

- Statt klassischer Barcodes (eindimensional) oder QR-Codes (zweidimensional) kommt bevorzugt ein Infusionsauftrags- oder Infusion Order-spezifischer, alphanumerischer Kurzbestätigungscode oder Short Verification Code (SVC) zur Anwendung, welcher von der Pflegekraft einfach vom Label des Medikationscontainers abgelesen und über das User Interface der Pumpe eingegeben werden kann.
- Mit bevorzugt lediglich drei alphanumerischen Zeichen (lateinische Großbuchstaben ohne "O" sowie Ziffern von 0 bis 9) bietet der SVC rund 42.000 Codevarianten, was spätestens bei einer Begrenzung der Gültigkeitsdauer ausreichen sollte, um für jede Infusion Order eine eindeutigen Code erstellen zu können. Damit kann bei der Verifikation des Medikationscontainers auf einen Scanner oder anderweitiges Lesegerät (z. B. Smart Device) verzichtet werden.

### VIA-bedingte Neuerungen auf Seiten des EMR-Systems

- Die Infusion Order enthält lediglich eine unspezifische, den VIA-Modus aktivierende Pumpen-ID, da der gesamte Prozess über die vom Benutzer ausgewählte Pumpe gesteuert wird und die Pumpe daher nicht mehr im Vorfeld aktiv adressiert werden muss.
- Für die Verifikation des Medikationscontainers wird ein eindeutiger, dreistelliger SVC mit erzeugt und der Infusion Order entweder zusätzlich oder an Stelle der im BCMA-Prozess vorgesehenen Order ID mitgegeben.
- Optional wird der Infusion Order eine früheste Startzeit und/oder eine begrenzte zeitliche Gültigkeit mitgegeben, welche vom empfangenden Infusionspumpen-System entsprechend auszuwerten ist.
- Denkbar ist auch eine räumliche bzw. logische Einschränkung, z. B. auf eine bestimmte Care Unit oder einen bestimmten Nutzerkreis (diensthabende Schwester, Schwestern der jeweiligen Station etc.).
- In diesem Fall muss müssen die zulässigen Ausprägungen zusätzlich im Infusion Order enthalten sein und ein zusätzlicher, pflegekraft-spezifischer SVC im Rahmen der Order Verifikation an der Pumpe eingegeben werden.

### VIA-bedingte Neuerungen im Infusionspumpen-System

- Sofern die vom EMR-System gesendete Infusion Order eine spezielle, den VIA-Modus auslösende Pumpen-ID enthält, wird sie in einer zusätzlichen Systemkomponente, dem Infusion Order Manager (IOM) in einer Art Infusion Order Backlog zwischengespeichert.
- An jeder Infusionspumpe lässt sich vom User aktiv die Pull-Kommunikation zum Abruf von für den Bettplatz bzw. Patienten vorliegenden Infusion Orders auslösen.
- Der Infusion Order Manager erkennt, wenn an einer Pumpe die Pull-Kommunikation ausgelöst wird / eine Pumpe in den Empfangsmodus für Infusion Order versetzt wird. Bei gegebener Bettplatz-Zuordnung der Pumpe sendet der IOM dann eine vorliegende, diesem Bettplatz zugeordnete Infusion Order automatisch an genau diese Pumpe.
- Nach Empfang der Infusion Order fordert die Pumpe den User zur Eingabe des Medikations-SVC auf und vergleicht ihn mit dem SVC, der in der vom IOM empfangenen Infusion Order enthalten ist. Bei erfolgreicher Prüfung werden sämtliche Daten des Infusion Orders auf dem Display der Pumpe zur finalen Bestätigung angezeigt, ansonsten wird der Vorgang mit entsprechender Fehlermeldung abgebrochen.
- Sofern dem Infusion Order Manager mehrere Infusion Orders für einen Bettplatz vorliegen, wird unter Berücksichtigung der gegebenenfalls gegebenen zeitlichen Einschränkungen (Früheste Startzeit noch nicht erreicht? Zulässiges Zeitfenster abgelaufen?) eine Listenauswahl dieser Orders generiert und auf der in Empfangsbereitschaft geschalteten Pumpe angezeigt. Nach Auswahl einer Infusion Order über das User Interface der Pumpe schickt der Infusion Order Manager diese Order an diese Pumpe. Werden an einer weitere, dem gleichen Bettplatz zugeordneten Pumpe die verfügbaren Infusionsaufträge abgerufen, wird die bereits an eine andere Pumpe geschickte Infusionsauftrag bestenfalls noch angezeigt, ist aber nicht mehr auswählbar.
- Die Liste enthält optional auch eine Lösch-Funktion für noch nicht gestartete Infusion Orders (gegebenenfalls nur ausführbar nach Eingabe eines temporär gültigen Lösch-SVC).

### Wünschenswerte Voraussetzungen

- Das EMR-System kann den Patienten zu jedem Zeitpunkt eindeutig einem Bettplatz zuordnen.
- Im Infusionspumpensystem lassen sich die Pumpen ebenfalls einem eindeutigen Bettplatz zuordnen.
- Bei Beschränkung auf einen dreistelligen alphanumerischen Code reichen die rund 42.000 möglichen Ausprägungen (ggfs. zusätzlich mit begrenzter Gültigkeitsdauer) aus, um die Anzahl der innerhalb dieser Gültigkeitsdauer gleichzeitig anfallenden Infusionen sicher abzubilden (abhängig von der Anzahl der Betten, Pumpen und Infusionen pro Pumpe).

### Typischer Ablauf der VIA-Programmierung

1. Das EMR-System sendet die Infusion Order für den Patienten X an das Infusionspumpen-System.
2. Die Krankenhausapotheke oder die Pflegekraft bereiten den entsprechenden Medikationscontainer vor.
3. Die Krankenhausapotheke oder die Pflegekraft versehen den entsprechenden Medikationscontainer mit einem Aufkleber, welcher den orderspezifischen Short Verification Code enthält.
4. Die Pflegekraft legt den Medikationscontainer in eine beliebige, dem aktuellen Bettplatz von Patient X zugeordnete Infusionspumpe und aktiviert die Pull-Kommunikation.
5. Die Infusionspumpe empfängt die Infusion Order und verlangt die Eingabe des Short Verification Code.
6. Alternativ: Die Pumpe empfängt die Liste der vorliegenden Infusion Orders und zeigt sie zur Auswahl auf dem Display. Nach entsprechender Auswahl empfängt die Pumpe die Order und verlangt die Eingabe des dazugehörigen Short Verification Code.
7. Die Pflegekraft gibt den Code ein, prüft die daraufhin angezeigten Order-Daten gegen die Anzeige auf dem User Interface des EMR-Systems (alternativ gegen einen Ausdruck des Medikationsauftrags) und startet die Infusion.

### Vorteile des VIA-Systemkonzeptes

- Kein Scanner oder Smart Device wird für die Programmierung der Pumpe benötigt.
- Anstatt drei Scanvorgänge an unterschiedlichen Stellen durchzuführen und über das User Interface des EMR-Systems zu steuern, muss die Pflegekraft lediglich einen einfachen, dreistelligen Code an der Pumpe eingeben.
- Das User Interface des EMR-Systems wird höchstens einmal für den finalen Abgleich zwischen Programmierung und Medikationsauftrag benötigt.
- Sowohl aus Sicht des EMR-Systems als auch aus Sicht des Krankenhauses ist eine Implementierung wesentlich einfacher und schneller umzusetzen.
- Bei Vorhandensein mehrerer Infusion Orders für einen Patienten können diese einzeln über jede Pumpe ausgewählt und ausgeführt werden, die dem Bettplatz dieses Patienten zugeordnet ist.
- Auch Infusion Orders für einen späteren Zeitpunkt können vom EMR-System an das Infusionspumpen-System gesendet und im vordefinierten Zeitfenster dann auf jeder Pumpe ausgewählt und ausgeführt werden, die dem Bettplatz des entsprechenden Patienten zugeordnet ist.
- Vorgegebene Ausführungsreihenfolgen von Infusion Orders können automatisch überwacht werden (z. B. Infusion Order B lässt sich erst auswählen, wenn Infusion Order A gestartet oder abgeschlossen wurde).
- Das VIA-Konzept basiert weiterhin auf dem bekannten und in einigen Teilen der Welt seit Jahren etablierten AutoProgramming/BCMA-Workflow unter vorteilhafter Nutzung von HL7-Kommunikation und IHE Transaction Profiles. Diese lassen sich einfach um die wenigen, für den VIA-Workflow zusätzlich benötigten Angaben ergänzen.

Ein Ausführungsbeispiel der Erfindung wird anhand der beigefügten Zeichnungen näher erläutert. Es zeigt:
- FIG. 1: eine schematische Übersicht über eine medizinische Einrichtung, in der Infusionen an Patienten verabreicht werden, wobei verschiedene Prozessabläufe durch Pfeile veranschaulicht sind, und
- FIG. 2: eine schematische Darstellung einer Infusionspumpe zur Verwendung in einer derartigen Einrichtung.

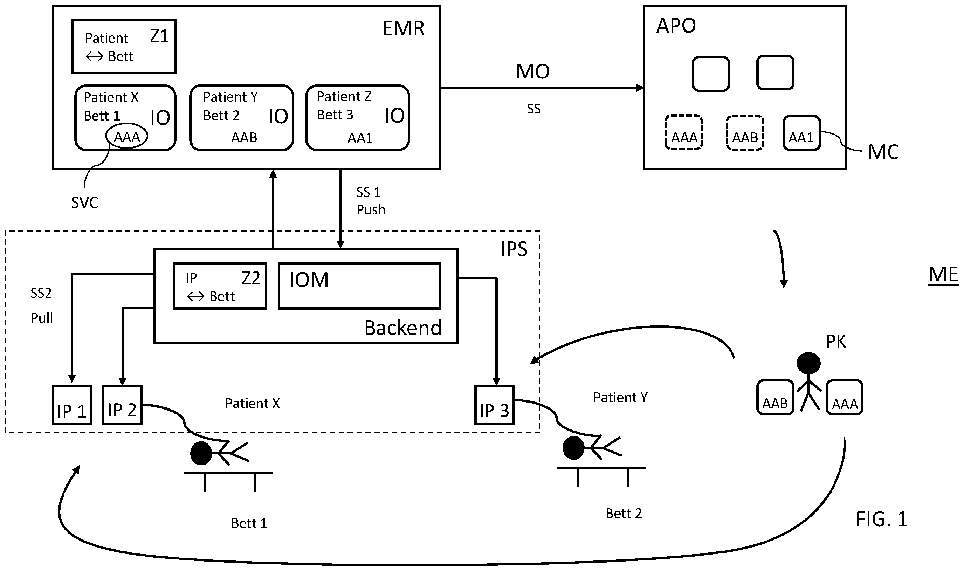

FIG. 1 gibt eine schematische Übersicht über eine medizinische Einrichtung ME, in der Infusionen an Patienten verabreicht werden. Dabei kann es sich beispielsweise um ein Krankenhaus oder ein Pflegeheim handeln. Die medizinische Einrichtung weist eine Mehrzahl von eindeutig identifizierbaren Betten auf, wobei hier im Beispiel die Identifizierung einfach über eine fortlaufende Bettnummer (Bett 1, Bett 2, ...) erfolgt. Im Allgemeinen kann die Identifizierung über eine Bettplatz-ID erfolgen. Jedes Bett kann mit einem Patienten belegt werden, der durch eine eindeutige Patienten-ID und/oder seine persönlichen Daten identifizierbar ist. Hier im Beispiel sind dies der Einfachheit halber Patient X, der Bett 1 belegt, und Patient Y, der Bett 2 belegt usw. Die aktuell gültige Zuordnung Z1 zwischen Betten und Patienten ist als Datensatz in einem elektronischen Patientendaten Management-System (engl. Electronic Medical Record-System) EMR hinterlegt.

Jedem der Betten ist zu einem beliebigen Zeitpunkt eine Anzahl von Infusionspumpen IP zugeordnet, wobei die Anzahl die Werte 0, 1, 2, ... annehmen kann. Das heißt, die möglichen Fälle "keine" oder "mehrere" Infusionspumpen IP sind enthalten, wobei jede der Infusionspumpen IP genau (und maximal) einem Bett zugeordnet ist. Die Infusionspumpen IP sind anhand einer Pumpen-ID eindeutig identifizierbar, hier im Beispiel in Gestalt einer fortlaufenden Nummerierung (IP 1, IP 2, ...). Im dargestellten aktuellen Zustand sind die Infusionspumpen IP 1 und IP 2 dem Bett 1 (und damit dem Patienten X) zugeordnet, während die Infusionspumpe IP 3 dem Bett 2 (und damit dem Patienten Y) zugeordnet ist. Die aktuelle Zuordnung Z2 zwischen Betten und Infusionspumpen ist als Datensatz in einem Backend eines Infusionspumpen-Systems IPS, welches die Infusionspumpen IP 1, IP 2, ... umfasst, hinterlegt.

Für eine effiziente und sichere Durchführung anstehender Infusionen ist eine vom EMR veranlasste und gesteuerte Fernprogrammierung der zugeordneten Infusionspumpe IP für den jeweiligen spezifischen Infusionsvorgang vorgesehen. Dazu generiert das EMR nach den Vorgaben der behandelnden Mediziner eine Anzahl von Infusionsaufträgen (engl. Infusion Orders) IO. Jeder Infusionsauftrag IO ist genau einem Patienten in dem von ihm belegten Bett zugeordnet und enthält Angaben zu dem zu verabreichenden Medikament bzw. zur Infusionslösung, zur Menge, zur Flussrate usw. Optional kann der Infusionsauftrag IO mit einem Gültigkeitszeitraum bzw. einer Gültigkeitsdauer versehen sein. Auch eine Verabreichungsreihenfolge im Falle mehrerer Infusionen für denselben Patienten kann optional in den Infusionsauftrag IO integriert sein.

Des Weiteren wird jedem Infusionsauftrag IO ein eindeutiger Kurzbestätigungscode (engl. Short Verification Code) SVC zugeordnet, bei dem es sich im Beispiel um einen dreistelligen alphanumerischen Code handelt. Beispielsweise kann jede der drei Stellen gewählt sein aus der Gesamtheit der Großbuchstaben A bis Z und den Ziffern von 0 bis 9, wobei zur Vermeidung von Verwechslungen vorzugsweise auf die Verwendung von O (und ggf. auch I) verzichtet wird. Dies ergibt beispielsweise (25+10)^3 = 42.875 verschiedene Kennzeichnungsmöglichkeiten, bei einem alternativ möglichen vierstelligen Code und/oder bei Zulassung von Kleinbuchstagen noch deutlich mehr. Hier im Beispiel ist dem Infusionsauftrag IO für den Patienten X im Bett 1 der SVC "AAA" zugeordnet, während der Infusionsauftrag IO für den Patienten Y im Bett 2 den SVC "AAB" trägt. Der SVC kann jeweils in den Infusionsauftrag IO integriert oder als separates Element zugeordnet bzw. beigefügt sein.

Aus den Infusionsaufträgen IO werden Medikationsaufträge (engl. Medication Orders) MO abgeleitet oder generiert, die über eine geeignete Schnittstelle SS, vorzugsweise elektronisch, an eine (Krankenhaus-) Apotheke APO oder an einen Medikamentenlieferant übermittelt werden. Im Beispiel fertigt die Apotheke APO die benötigten Infusionslösungen gemäß den individuellen Vorgaben hinsichtlich Zusammensetzung, Menge usw. an und/oder stellt sie in Form verabreichungsbereiter Beutel, Spritzen oder dergleichen, die zusammenfassend auch als Medikationscontainer MC bezeichnet werden, bereit.

Bei der Übermittlung des Medikationsauftrags MO wird auch der zugeordnete Kurzbestätigungscode SVC übermittelt und dem jeweiligen Medikationscontainer als Beschriftung (Label) zugeordnet, beispielsweise durch Anbringung eines entsprechend bedruckten Aufklebers oder Etikettes. Im Beispiel sieht man unter anderem die mit den Kurzbestätigungscodes "AAA" und "AAB" beschrifteten Medikationscontainer MC, die in eindeutiger Weise den oben besprochen Infusionsaufträgen IO zugeordnet sind. Neben dem Kurzbestätigungscode SVC kann die Beschriftung des Medikationscontainers MC auch noch andere Angaben umfassen, etwa solche, die im zugeordneten Infusionsauftrag IO enthalten sind.

Das Patientendaten Management-System EMR übermittelt die einzelnen Infusionsaufträge IO unmittelbar nach deren Erstellung über eine geeignete elektronische Schnittstelle SS 1 mittels Push-Kommunikation an das Backend des Infusionspumpensystems IPS, wo sie in einem als Infusionsauftragsmanager (engl. Infusion Order Manager) IOM bezeichneten Programmmodul abgelegt werden. Dort stehen die übermittelten Infusionsaufträge IO zum Abruf durch die Infusionspumpen IP bereit, wie weiter unten näher erläutert wird.

Eine Pflegekraft PK oder ein sonstiger Benutzer entnimmt einer Benutzerschnittstelle des EMR beispielsweise die Information, dass für den Patienten X im Bett 1 ein Infusionsauftrag IO (mit dem Kurzbestätigungscode "AAA") bereitsteht, und entsprechend für den Patienten Y im Bett 2 (mit dem Kurzbestätigungscode "AAB"). Er/sie holt die den Infusionsaufträgen IO zugeordneten und entsprechend beschrifteten Medikationscontainer MC aus der Apotheke APO ab und bringt sie nacheinander zu den zugeordneten Betten und somit den zugeordneten Patienten. Beispielsweise weiß oder sieht die Pflegekraft, dass der Medikationscontainer MC mit dem SVC "AAA" dem Patienten X im Bett 1 zugeordnet ist. Dort stehen die beiden Infusionspumpen IP 1 und IP 2 bereit, und sofern beide nicht belegt sind, hat die Pflegekraft im Regelfall die freie Wahl zwischen beiden. Beispielsweise entscheidet sie sich für die Infusionspumpe IP 1 zur Durchführung der Infusion am Bett 1. In anderen Fällen mag die Wahlfreiheit durch eine schon vorhandene Belegung und/oder durch die Anzahl der Infusionspumpen am Bett und/oder durch weitere Kriterien eingeschränkt sein. Beispielsweise steht am Bett 2 nur die Infusionspumpe IP 3 zur Durchführung des Infusionsauftrags IO mit dem SVC "AAB" zur Verfügung.

Beispielsweise legt die Pflegekraft PK den Medikationscontainer MC mit dem SVC "AAA" in die Aufnahme der Infusionspumpe IP 1 am Bett 1 ein und aktiviert durch eine Eingabe an einem Eingabefeld (Tastatur oder Touch-Display) eine Pull-Kommunikation, bei der sich die Infusionspumpe über eine Schnittstelle SS 2 vom Backend des Infusionspumpensystems IPS einen ihr zugeordneten Infusionsauftrag IO abholt. Beispielsweise teilt dazu die Infusionspumpe IP 1 ihre Pumpen-ID dem Backend mit. Anhand der bekannten Zuordnung Z2 zwischen Infusionspumpen und Betten erkennt das Backend die Zugehörigkeit der Infusionspumpe IP 1 zum Bett 1 und überprüft das Vorhandensein von dem Bett 1 zugeordneten Infusionsaufträgen IO im Infusionsauftragsmanager IOM. Im Erfolgsfall wird der entsprechende Infusionsauftrag IO (hier mit dem SVC "AAA"), alternativ erst einmal nur eine ihm zugeordnete ID, über die elektronische Schnittstelle SS 2 vom Backend zur Infusionspumpe (hier IP 1) übermittelt.

Zur Verifikation, dass der richtige Medikationscontainer MC in die Aufnahme der Infusionspumpe IP eingelegt wurde, wird die Pflegekraft PK über ein Display an der Infusionspumpe IP aufgefordert, über das Eingabefeld den SVC, den sie vom Medikationscontainer MC abliest, einzugeben. Bei korrekter Eingabe (hier "AAA") wird die Infusionspumpe IP entsprechend dem vom Backend erhaltenen bzw. empfangenen Infusionsauftrag IO programmiert bzw. ein bereits zuvor erhaltener Infusionsauftrag IO oder eine erfolgte Programmierung wird zur Ausführung freigegeben. Andernfalls wird eine Fehlermeldung ausgegeben.

Falls im genannten Beispiel mehrere Infusionsaufträge für das Bett 1 anstehen, kann das Verfahren so ablaufen, dass am Display der verwendeten Infusionspumpe IP eine Auswahlliste der Aufträge, ggf. in Kombination mit einer Reihenfolge, angezeigt wird, woraufhin der Benutzer bzw. die Pflegkraft eine Auswahl vornimmt und im Anschluss - wie zuvor beschrieben - durch Eingabe auf dem Medikationscontainer befindlichen SVC die korrekte Medikation verifiziert und den Infusionsauftrag damit zur Ausführung freigibt.

In einer Abwandlung des Verfahrens kann vorgesehen sein, dass der Benutzer direkt beim oder nach dem Einlegen des Medikationscontainers MC in die Aufnahme den zugehörigen SVC im Eingabefeld der Infusionspumpe IP eingibt und dadurch veranlasst, dass die Infusionspumpe IP beim Backend nachfragt, ob dort ein diesem SVC zugeordneter Infusionsauftrag IO hinterlegt ist. Im Erfolgsfall "zieht" sich die Infusionspumpe IP eben jenen Infusionsauftrag aus dem IOM im Backend. Die weiter oben beschriebene Auswahl aus einer Liste offener Medikationsaufträge wäre in diesem Fall jedoch nicht möglich, da die Auswahl durch Eingabe des SVC bereits erfolgt ist - insofern könnte die Liste der weiteren Aufträge für diesen Patienten hier lediglich zur Information angezeigt werden.

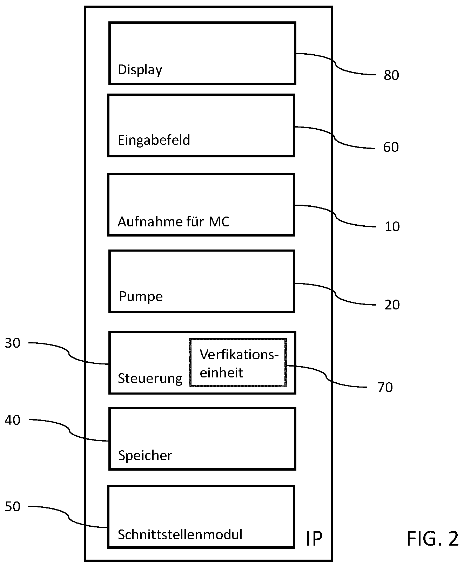

Eine Infusionspumpe IP zur Verwendung in dem beschriebenen Szenario ist in FIG. 2 schematisch dargestellt. Die Infusionspumpe IP weist eine Aufnahme 10 für einen Medikationscontainer MC auf und eine an den Medikationscontainer MC anschließbare Pumpe 20, die durch einen integrierte Steuerung 30 angesteuert wird. Ein individuell auf den Medikationscontainer MC und den Behandlungsfall abgestimmter Infusionsauftrag IO ist nach Art eines Steuerprogramms in einem beschreibbaren Speicher 40 hinterlegt bzw. durch Hineinladen hinterlegbar. Der Infusionsauftrag IO kann über ein Schnittstellenmodul 50 als Bestandteil der Schnittstelle SS 2 per "Pull" Kommunikation von einem Backend eines Infusionspumpensystems IPS empfangen werden. Ein zugehöriger, auf dem Medikationscontainer MC aufgedruckter bzw. als Label beigefügter Kurzbestätigungscode SVC wird dazu von einer Pflegekraft PK abgelesen, über ein Eingabefeld 60 eingegeben und dann von einer Verifikationseinheit 70 der Steuerung 30 mit dem unmittelbar dem Infusionsauftrag IO zugeordneten Kurzbestätigungscode SVC verglichen und bei Übereinstimmung verifiziert. Das Eingabefeld 60 kann durch ein berührungsempfindliches Tastenfeld auf einem Touch-Display, welches der Benutzerführung und der Ausgabe des Verifikationsergebnisses dient, realisiert sein. In diesem Fall kann das Tastenfeld speziell für die Eingabe des SVC optimiert sein. Alternativ kann ein separates Display 80 vorgesehen sein.

Zusammenfassend wird durch die beschriebene Verwendung und Abfrage des Kurzbestätigungscode SVC auf überraschend einfache Weise eine Verwechslung beim Transport der Medikationscontainer MC von der Apotheke oder vom Lieferanten zum zugeordneten Patientenbett vermieden, ohne dass es umständlicher Scanvorgänge oder dergleichen bedarf.

### Bezugszeichenliste

- APO: Apotheke
- Backend: Backend
- Bett: Patientenbett
- EMR: elektronisches Patientendaten Management-System
- IO: Infusionsauftrag
- IOM: Infusionsauftragsmanager
- IP: Infusionspumpe
- IPS: Infusionspumpensystem
- MC: Medikationscontainer
- ME: Medizinische Einrichtung
- MO: Medikationsauftrag
- PK: Pflegekraft
- SVC: Kurzbestätigungscode
- SS: Schnittstelle
- Z: Zuordnung

- 10: Aufnahme
- 20: Pumpe
- 30: Steuerung
- 40: Speicher
- 50: Schnittstellenmodul
- 60: Eingabefeld
- 70: Verifikationseinheit
- 80: Display

## Patentansprüche

1. Verfahren zur automatischen Fernprogrammierung von Infusionspumpen (IP) mittels Infusionsaufträgen (IO), die in/von einem Patientendaten Management-System (EMR) einer medizinischen Einrichtung (ME) bereitgestellt werden, wobei die medizinische Einrichtung (ME)
• eine Anzahl von Betten aufweist, die jeweils mit einem Patienten belegbar sind,
• eine Anzahl von Infusionspumpen (IP) aufweist, die jeweils an ein Backend eines Infusionspumpensystems (IPS) angeschlossen sind,
wobei
• das Patientendaten Management-System (EMR) über eine erste elektronische Schnittstelle (SS 1) Infusionsaufträge (IO) zur Zwischenspeicherung an das Backend übermittelt,
• die jeweilige Infusionspumpe (IP) über eine zweite elektronische Schnittstelle (SS 2) zwischengespeicherte Infusionsaufträge (IO) vom Backend empfängt,
wobei ferner
• dem jeweiligen Infusionsauftrag (IO) ein von einem Menschen lesbarer Kurzbestätigungscode (SVC) als eindeutige Kennung unmittelbar zugeordnet und bei Übermittlungen mitgegeben wird,
• dem jeweiligen Infusionsauftrag (IO) ein Medikationscontainer (MC) zugeordnet wird, der mit einem den zugeordneten Kurzbestätigungscode (SVC) enthaltenden Informationsträger ausgestattet oder verbunden ist,
• die jeweilige Infusionspumpe (IP) eine Aufnahme (10) oder einen Anschluss für einen der Medikationscontainer (MC) aufweist,
• der jeweiligen Infusionspumpe (IP) ein Eingabemittel, insbesondere ein an der Infusionspumpe (IP) angebrachtes Eingabefeld (60), zur Eingabe des zugehörigen, dem Informationsträger des Medikationscontainers (MC) entnehmbaren Kurzbestätigungscodes (SVC) zugeordnet ist,
wobei schließlich
in einem Verifikationsschritt ein am Eingabemittel eingegebener Kurzbestätigungscode (SVC) mit dem Kurzbestätigungscode (SVC), der dem von der Infusionspumpe (IP) empfangenen Infusionsauftrag (IO) unmittelbar zugeordnet ist, verglichen wird, und wobei nur bei Übereinstimmung der Kurzbestätigungscodes (SVC) der Infusionsauftrag (IO) von der Infusionspumpe (IP) ausgeführt wird oder auf Aufforderung ausführbar ist.

2. Verfahren nach Anspruch 1, wobei das Patientendaten Management-System (EMR) eine Zuordnung (Z1) von Betten zu Patienten enthält.

3. Verfahren nach Anspruch 1 oder 2, wobei das Backend eine Zuordnung (Z2) von Betten zu Infusionspumpen enthält.

4. Verfahren nach einem der vorangehenden Ansprüche, wobei der Vergleich der Kurzbestätigungscodes (SVC) in einer in die jeweilige Infusionspumpe (IP) integrierten Steuerung (30) erfolgt.

5. Verfahren nach einem der vorangehenden Ansprüche, wobei die jeweilige Infusionspumpe (IP) via Pull-Kommunikation über die zweite elektronische Schnittstelle (SS 2) ihr indirekt zugeordnete Infusionsaufträge vom Backend abfragt.

6. Verfahren nach einem der vorangehenden Ansprüche, wobei das Patientendaten Management-System (EMR) Infusionsaufträge (IO) via Push-Kommunikation über die erste elektronische Schnittstelle (SS 1) an das Backend übermittelt.

7. Verfahren nach einem der vorangehenden Ansprüche, wobei dem jeweiligen Infusionsauftrag (IO) ein Gültigkeitszeitraum oder eine Gültigkeitsdauer zugeordnet und im Verifikationsschritt berücksichtigt wird.

8. Verfahren nach einem der vorangehenden Ansprüche, wobei der Kurzbestätigungsode (SVC) ein alphanumerischer Code ist.

9. Verfahren nach einem der vorangehenden Ansprüche, wobei der der Medikationscontainer (MC) mit dem zugeordneten Kurzbestätigungscode (SVC) beschriftet oder gekennzeichnet ist.

10. System zur automatischen Fernprogrammierung von Infusionspumpen (IP) mittels Infusionsaufträgen (IO), die in einem Patientendaten Management-System (EMR) einer medizinischen Einrichtung (ME) bereitgestellt werden, wobei die medizinische Einrichtung (ME)
• eine Anzahl von Betten aufweist, die jeweils mit einem Patienten belegbar sind,
• eine Anzahl von Infusionspumpen (IP) aufweist, die jeweils an ein Backend eines Infusionspumpensystems (IPS) angeschlossen sind,
wobei
• das Patientendaten Management-System (EMR) dazu ausgelegt oder programmiert ist über eine erste elektronische Schnittstelle (SS 1) Infusionsaufträge (IO) zur Zwischenspeicherung an das Backend zu übermitteln,
• die jeweilige Infusionspumpe (IP) dazu ausgelegt oder programmiert ist, über eine zweite elektronische Schnittstelle (SS 2) zwischengespeicherte Infusionsaufträge (IO) vom Backend zu empfangen,
wobei ferner
• dem jeweiligen Infusionsauftrag (IO) ein von einem Menschen lesbarer Kurzbestätigungscode (SVC) als eindeutige Kennung unmittelbar zugeordnet ist,
• dem jeweiligen Infusionsauftrag (IO) ein Medikationscontainer (MC) zugeordnet ist, der mit einem den zugeordneten Kurzbestätigungscode (SVC) enthaltenden Informationsträger ausgestattet oder verbunden ist,
• die jeweilige Infusionspumpe (IP) eine Aufnahme (10) oder einen Anschluss für einen der Medikationscontainer (MC) aufweist,
• der jeweiligen Infusionspumpe (IP) ein Eingabemittel, insbesondere ein an der Infusionspumpe (IP) angebrachtes Eingabefeld (60), zur Eingabe des zugehörigen, dem Informationsträger des Medikationscontainers (MC) entnehmbaren Kurzbestätigungscodes (SVC) zugeordnet ist,
wobei schließlich
das System eine Verifikationseinheit (70) umfasst, die dazu ausgelegt oder programmiert ist, einen am Eingabemittel eingegebenen Kurzbestätigungscode (SVC) mit dem Kurzbestätigungscode (SVC), der dem von der Infusionspumpe (IP) empfangenen Infusionsauftrag (SVC) unmittelbar zugeordnet ist, zu vergleichen, so dass nur bei Übereinstimmung der Kurzbestätigungscodes (SVC) der Infusionsauftrag (IO) von der Infusionspumpe (IP) ausgeführt wird oder auf Aufforderung ausführbar ist.
